# EUROPEAN PATENT SPECIFICATION

(11) **EP 1 545 552 B1**
(45) Date of publication and mention of the grant of the patent: **28.03.2007**
(21) Application number: 03729907.0
(22) Date of filing: 19.06.2003
(51) Int. Cl.: A61K 31/662, A61K 31/343, A61P 25/00

(54) **COMBINATION THERAPY WHEREIN A SEROTONIN REUPTAKE INHIBITOR IS USED**
KOMBINATIONSTHERAPIE MIT VERWENDUNG EINES SEROTONIN-WIEDERAUFNAHMEHEMMERS
THERAPIE D'ASSOCIATION FAISANT APPEL A UN INHIBITEUR DU RECAPTAGE DE LA SEROTONINE

(30) Priority: 20.06.2002 DK 200200943; 20.06.2002 US 390851 P
(43) Date of publication of application: 29.06.2005
(73) Proprietor: H. LUNDBECK A/S, 2500 Valby-Copenhagen (DK)
(72) Inventor: MORK, Arne, DK-2760 Malov (DK); CREMERS, Thomas, Ivo, Franciscus, Hubert, NL-9717 KV Groningen (NL); WILLIGERS, Sandra, DK-2880 Bagsvaerd (DK)
(74) Representative: Kjerrumgaard, Lars Bo
(86) International application number: PCT/DK2003/000412
(87) International publication number: WO 2004/000326

(56) References cited:
- WO-A1-99/37303
- LONDBORG M.D. PETER D. ET AL.: 'Short-term cotherapy with clonazepam and fluoxetine: anxiety, sleep disturbance and core symptoms of depression' JOURNAL OF AFFECTIVE DISORDERS vol. 61, 2000, pages 73 - 79, XP002962665

## Description

The present invention relates to the combination of a serotonin reuptake inhibitor and a GABA_{B} receptor antagonist. Accordingly, the present invention relates to the use of certain compounds, and to compositions of compounds having serotonin reuptake inhibiting activity and GABA_{B} antagonistic, partial agonistic or inverse agonistic activity for the manufacture of a medicament for the treatment of depression and other affective disorders. The combined serotonin reuptake inhibiting effect and the GABA_{B} antagonistic, partial agonistic or inverse agonistic effect may reside within the same chemical entity or in two separate chemical entities.

### Background

Selective serotonin reuptake inhibitors (hereinafter referred to as SSRIs) have become first choice therapeutics in the treatment of depression, certain forms of anxiety and social phobias, because they are effective, well tolerated and have a favourable safety profile compared to the classic tricyclic antidepressants.

However, clinical studies on depression and anxiety disorders indicate that non-response to SSRIs is substantial, up to 30%. Another, often neglected, factor in antidepressant treatment is compliance, which has a rather profound effect on the patient's motivation to continue pharmacotherapy.

First of all, there is the delay in therapeutic effect of SSRIs. Sometimes symptoms even worsen during the first weeks of treatment. Secondly, sexual dysfunction is a side effect common to all SSRIs. Without addressing these problems, real progress in the pharmacotherapy of depression and anxiety disorders is not likely to happen.

In order to cope with non-response, psychiatrists sometimes make use of augmentation strategies. Augmentation of antidepressant therapy may be accomplished through the co-administration of mood stabilizers such as lithium carbonate or triiodothyronin or by the use of electroshock.

In 1993, an augmentation strategy with pindolol was described by Artigas et al. in *Trends Pharmacol. Sci.* **1993**, *14,* p 262-263. Artigas' idea is based on intracerebral microdialysis experiments in animals. In fact, later neurochemical studies built on the desensitization hypothesis by Blier and co-workers stated that the delay in therapeutic effect of antidepressants is related to a gradual desensitization of 5-HT autoreceptors (Blier et al. *J. Clin. Psycipharmacol.* **1987,** *7 suppl.* 6, 24S-35S). A key point in their hypothesis is that the effects of SSRIs on the release-controlling somatodendritic autoreceptors (5-HT_{1A}) limit the release of 5-HT in terminal areas and thus the effect of 5-HT uptake inhibition in those regions. This is supported by microdialysis experiments in rats, showing that the increase in extracellular 5-HT elicited by a single dose of an SSRI is augmented by co-administration of a 5-HT_{1A} autoreceptor antagonist (Invernizzi et al. *Brain Res,* **1992,** *584,* p 322-324 and Hjorth, S., J. *Neurochem,* **1993,** *60*, p 776-779).

The effect of combined administration of a compound that inhibits serotonin reuptake and a 5-HT_{1A} receptor antagonist has been evaluated in several studies (Innis, R.B. et al. *Eur. J. Pharmacol.* **1987,** *143,* p. 1095-204 and Gartside, S.E., *Br. J. Pharmacol,* **1995,** *115*, p 1064-1070, Blier, P. et al. *Trends in Pharmacol. Science* **1994,** *15*, 220). In these studies it was found that 5-HT_{1A} receptor antagonists would abolish the initial brake on 5-HT neurotransmission induced by the serotonin reuptake inhibitors and thus produce an immediate boost of 5-HT transmission and a rapid onset of therapeutic action.

Several patent applications have been filed which cover the use of a combination of a 5-HT_{1A} antagonist and a serotonin reuptake inhibitor for the treatment of depression (see e.g. EP-A2-687 472 and EP-A2-714 663).

Another approach to increase terminal 5-HT would be through blockade of the 5-HT_{1B} autoreceptor. Microdialysis experiments in rats have indeed shown that increase of hippocampal 5-HT by citalopram is potentiated by GMC 2-29, an experimental 5-HT_{1B} receptor antagonist.

Several patent applications covering the combination of an SSRI and a 5-HT_{1B} antagonist or partial agonist have also been filed (WO 97/28141, WO 96/03400, EP-A-701819 and WO 99/13877).

γ-aminobutyric acid (GABA) is the most important inhibitory neurotransmitter in the brain; up to 50% of all central synapses are GABA-ergic (Paredes R.G. & Agmo A., Neuroscience and Biobehavioural Reviews, vol 16: pp 145-170 (1992)).

Noradrenaline, dopamine and serotonin (5-HT) are all under inhibitory control of GABA (Haefely W. The role of GABA in anxiolytic/antidepressant drug action. Elliott M.M., Heal D.J. & Marsden C.A. (eds), pp 151-168, John Wiley & Sons, New York (1992)). There are two subtypes of GABA receptors, GABA_{A} and GABA_{B}, which have been extensively studied and their influence on 5HT nerve function and release have been performed.

Thus stimulation of the GABA_{A} receptor with the agonist muscimol reduced 5-HT cell activity & 5-HT release in the raphé nuclei (Tao R. & Auerbach S.B. J. Psychopharmacology vol 14(2): pp 100-113 (2000)) and blockade of GABA_{A} receptors increases firing and subsequently elevates levels of extracellular 5-HT (see below - bicuculline & Tao R. *et al.,* British Journal of Pharmacology vol 119: pp 1375-1384 (1996)).

The GABA_{B} agonist baclofen produced a decrease in 5-HT in the raphé and reduction in the forebrain (Tao R. *et al.,* British Journal of Pharmacology vol 119: pp 1375-1384 (1996)) and when administered by itself the GABA_{B} antagonist, phaclofen, is devoid of effect on 5-HT levels in either the raphé (Abellán M.T. *et al.,* Neuroreport vol 11: pp941-945 (2000); Tao R. *et al.,* British Journal of Pharmacology vol 119: pp 1375-1384 (1996)) or in the forebrain (see below & Tao R. *et al.,* British Journal of Pharmacology vol 119: pp 1375-1384 (1996)). However, phaclofen, administered either centrally into the hippocampus or systemically was shown to significantly increase the effects of citalopram on extracellular 5-HT levels, as demonstrated in the findings reported here.

### The invention

It has now surprisingly been found that a GABA_{B} antagonist will augment the effect of an SSRI on extracellular 5-HT levels.

It is therefore suggested that the combination of an SSRI and a GABA_{B} antagonist or a molecule, which has both 5-HT reuptake inhibitory and GABA_{B} antagonistic properties, would have a better efficacy and faster onset than an SSRI alone.

Antagonism at any GABA_{B} splice variants, including but not limited to GABA_{BR1a} and GABA_{BR1b} is claimed.

This invention covers both SSRI + GABA_{B} antagonist in separate or the same molecule.

### The present invention thus provides:

The use of a GABA_{B} receptor antagonist, inverse agonist or partial agonist for the preparation of a pharmaceutical composition to be used in combination with a serotonin reuptake inhibitor (SRI).

The present invention relates to the use of a compound, which is a serotonin reuptake inhibitor, and another compound, which is a GABA_{B} receptor antagonist, inverse agonist or partial agonist for the preparation of a pharmaceutical composition for the treatment of depression, anxiety disorders and other affective disorders, such as generalized anxiety disorder, panic anxiety, obsessive compulsive disorder, acute stress disorder, post traumatic stress disorder and social anxiety disorder, eating disorders such as bulimia, anorexia and obesity, phobias, dysthymia, premenstrual syndrome, cognitive disorders, impulse control disorders, attention deficit hyperactivity disorder, drug abuse or any other disorder responsive to serotonin reuptake inhibitors.

The present invention also relates to the use of a GABA_{B} receptor antagonist, inverse agonist or partial agonist for the preparation of a pharmaceutical composition useful for augmenting and/or providing faster onset of the therapeutic effect of a serotonin reuptake inhibitor.

In a preferred embodiment, the invention relates to the use as above wherein the serotonin reuptake inhibitor is used for the treatment of depression, anxiety disorders and other affective disorders, including generalized anxiety disorder, panic anxiety, obsessive compulsive disorder, acute stress disorder, post traumatic stress disorder or social anxiety disorder, eating disorders such as bulimia, anorexia and obesity, phobias, dysthymia, premenstrual syndrome, cognitive disorders, impulse control disorders, attention deficit hyperactivity disorder, drug abuse and any other disorder responsive to a SRI.

In another embodiment, the invention relates to the use of
a) a compound which is a serotonin reuptake inhibitor and a GABA_{B} receptor antagonist, inverse agonist or partial agonist, or
b) a combination of a compound, which is a serotonin reuptake inhibitor, and a compound, which is a GABA_{B} receptor antagonist, inverse agonist or partial agonist,
for the preparation of a pharmaceutical composition or kit (kit-of-parts) useful for the treatment of depression, anxiety disorders and other affective disorders, such as generalized anxiety disorder, panic anxiety, obsessive compulsive disorder, acute stress disorder, post traumatic stress disorder and social anxiety disorder, eating disorders such as bulimia, anorexia and obesity, phobias, dysthymia, premenstrual syndrome, cognitive disorders, impulse control disorders, attention deficit hyperactivity disorder, drug abuse or any other disorder responsive to serotonin reuptake inhibitors.

In two independent embodiments, the invention relates to the use of a compound, which is a serotonin reuptake inhibitor, and a compound, which is a GABA_{B} receptor antagonist, inverse agonist or partial agonist, for the preparation of a:
(a) pharmaceutical composition, or
(b) kit
useful for the treatment of depression, anxiety disorders and other affective disorders, such as generalized anxiety disorder, panic anxiety, obsessive compulsive disorder, acute stress disorder, post traumatic stress disorder and social anxiety disorder, eating disorders such as bulimia, anorexia and obesity, phobias, dysthymia, premenstrual syndrome, cognitive disorders, impulse control disorders, attention deficit hyperactivity disorder, drug abuse or any other disorder responsive to serotonin reuptake inhibitors.

In a further embodiment, the invention relates to a pharmaceutical composition or kit comprising:
a) a compound, which is a serotonin reuptake inhibitor, and a GABA_{B} receptor antagonist, inverse agonist or partial agonist, or
b) a combination of a compound, which is a serotonin reuptake inhibitor, and another compound, which is a GABA_{B} receptor antagonist, inverse agonist or partial agonist,
and optionally pharmaceutically acceptable carriers or diluents.

In two further individual embodiments, the invention relates to either a pharmaceutical composition or a kit comprising a compound, which is a serotonin reuptake inhibitor, and another compound, which is a GABA_{B} receptor antagonist, inverse agonist or partial agonist, and optionally pharmaceutically acceptable carriers or diluents.

In yet another embodiment, the invention relates to a method for the treatment of depression, anxiety disorders and other affective disorders, such as generalized anxiety disorder, panic anxiety, obsessive compulsive disorder, acute stress disorder, post traumatic stress disorder and social anxiety disorder, eating disorders such as bulimia, anorexia and obesity, phobias, dysthymia, premenstrual syndrome, cognitive disorders, impulse control disorders, attention deficit hyperactivity disorder, drug abuse or any other disorder responsive to serotonin reuptake inhibitors comprising administering to a person in need thereof a therapeutically effective amount of
a) a compound, which is a serotonin reuptake inhibitor, and a GABA_{B} receptor antagonist, inverse agonist or partial agonist, or
b) a combination of a compound, which is a serotonin reuptake inhibitor and a compound, which is a GABA_{B} receptor antagonist, inverse agonist or partial agonist.

Whenever mentioned, each of the options
a) a compound, which is a serotonin reuptake inhibitor, and a GABA_{B} receptor antagonist, inverse agonist or partial agonist, and
b) a combination of a compound, which is a serotonin reuptake inhibitor and a (or another) compound, which is a GABA_{B} receptor antagonist, inverse agonist or partial agonist
are intended to be individual embodiments. Accordingly, each of them may be claimed individually.

Each of the medical indications depression, anxiety disorders and other affective disorders, including generalized anxiety disorder, panic anxiety, obsessive compulsive disorder, acute stress disorder, post traumatic stress disorder or social anxiety disorder, eating disorders such as bulimia, anorexia and obesity, phobias, dysthymia, premenstrual syndrome, cognitive disorders, impulse control disorders, attention deficit hyperactivity disorder, drug abuse and any other disorder responsive to a SRI is intended to be an individual embodiment. Accordingly, whenever mentioned in the present description, each of the indications specified above may be claimed individually.

Whenever the indications depression, anxiety disorders and other affective disorders, including generalized anxiety disorder, panic anxiety, obsessive compulsive disorder, acute stress disorder, post traumatic stress disorder or social anxiety disorder, eating disorders such as bulimia, anorexia and obesity, phobias, dysthymia, premenstrual syndrome, cognitive disorders, impulse control disorders, attention deficit hyperactivity disorder, drug abuse and any other disorder responsive to a SRI are mentioned in relation to use of a GABA_{B} receptor antagonist, inverse agonist or partial agonist and an SRI, a pharmaceutical composition, a kit, a method of treatment and a method for the identification of compounds useful for treatment it is intended to be an individual embodiment. Accordingly, each of the indications specified above may individually be claimed together with said use of a GABA_{B} receptor antagonist, inverse agonist or partial agonist and an SRI, pharmaceutical composition, kit, method of treatment and method for the identification of compounds useful for treatment.

In a particular embodiment, a selective serotonin reuptake inhibitor is used according to the invention.

In another particular embodiment, a compound, which is selective for the GABA_{B} receptor is used according to the invention.

In a further embodiment, a compound, which is an antagonist, an inverse agonist at the GABA_{B} receptor is used according to the invention.

The pharmaceutical composition or kit according to the invention may be administered by simultaneous administration. The term "simultaneous administration" as used herein means, that the GABA_{B} receptor antagonist, inverse agonist or partial agonist and the SRI are administered with a time separation of no more than 15 minutes, such as at most 10 minutes, such as at most 5 minutes or such as at most 2 minutes. The GABA_{B} receptor antagonist, inverse agonist or partial agonist and the SRI may be contained in the "same unit dosage form" or in "discrete dosage forms". As used herein, the term "same unit dosage form" means a dosage form comprising both the SRI and the GABA_{B} receptor antagonist, inverse agonist or partial agonist. As used herein, the term "discrete dosage form" means that the GABA_{B} receptor antagonist, inverse agonist or partial agonist is comprised in one dosage form and that the SRI is comprised in another dosage form.

Simultaneous administration of GABA_{B} receptor antagonist, inverse agonist or partial agonist and the SRI is optionally combined with administration of supplementary doses of GABA_{B} receptor antagonist, inverse agonist or partial agonist. The supplementary doses of GABA_{B} receptor antagonist, inverse agonist or partial agonist may be given for instance 1, 2, 3 or 4 times a day whereas the SRI and the GABA_{B} receptor antagonist, inverse agonist or partial agonist which are administered by "simultaneous administration" may be given one or more times a day, e.g. once daily or e.g. twice daily. Accordingly:
- the GABA_{B} receptor antagonist, inverse agonist or partial agonist and the SRI may be administered by simultaneous administration once daily and supplementary doses of GABA_{B} receptor antagonist, inverse agonist or partial agonist may be administered 1, 2, 3 or 4 times a day, such as 1, 2 or 3 times a day, such as once or twice daily, such as twice daily or such as once daily,
   or
- the GABA_{B} receptor antagonist, inverse agonist or partial agonist and the SRI may be administered by simultaneous administration twice daily and supplementary doses of GABA_{B} receptor antagonist, inverse agonist or partial agonist may be administered 1, 2, 3 or 4 times a day, such as 1, 2 or 3 times a day, such as once or twice daily, such as twice daily or such as once daily.

Alternatively, the pharmaceutical composition or kit according to the invention is administered by sequential administration. The term "sequential administration" as used herein means that 1 or more daily doses of GABA_{B} receptor antagonist, inverse agonist or partial agonist and 1 or more daily doses of SRI are administered with a time separation between two administered doses of more than 15 minutes and less than 4 hours, such as more than 2 hours and less than 4 hours, such as more than 15 minutes and less than 2 hours, such as more than 1 hour and less than 2 hours, such as more than 30 minutes and less than 1 hour, such as more than 15 minutes and less than 30 minutes. Either the SRI or the GABA_{B} receptor antagonist, inverse agonist or partial agonist may be administered first. The GABA_{B} receptor antagonist, inverse agonist or partial agonist and the SRI are contained in discrete dosage forms, optionally contained in the same container or package. Typically, 1, 2, 3, 4 or 5 daily doses of GABA_{B} receptor antagonist, inverse agonist or partial agonist and 1 or 2 daily doses of SRI may be administered. Accordingly:
- the GABA_{B} receptor antagonist, inverse agonist or partial agonist and the SRI may be administered once daily and the GABA_{B} receptor antagonist, inverse agonist or partial agonist may be administered 1, 2, 3, 4 or 5 times a day, such as 1, 2, 3 or 4 times a day, such as 1, 2 or 3 times a day, such as once or twice daily, such as twice daily or such as once daily,
   or
- the GABA_{B} receptor antagonist, inverse agonist or partial agonist and the SRI may be administered twice daily and the GABA_{B} receptor antagonist, inverse agonist or partial agonist may be administered 1, 2, 3, 4 or 5 times a day, such as 1, 2, 3 or 4 times a day, such as 1, 2 or 3 times a day, such as once or twice daily, such as twice daily or such as once daily.

Accordingly, the pharmaceutical composition or kit according to the invention may be adapted for simultaneous administration of the active ingredients, or it may be adapted for sequential administration of the active ingredients. When the pharmaceutical composition or kit is adapted for simultaneous administration, the active ingredients may be contained in the same unit dosage form. When the pharmaceutical composition or kit is adapted for sequential administration, the active ingredients are contained in discrete dosage forms, optionally contained in the same container or package. As used herein, an "active ingredient" means an SRI or a GABA_{B} receptor antagonist, inverse agonist or partial agonist.

A kit (kit-of-parts) comprises a preparation of the GABA_{B} receptor antagonist, inverse agonist or partial agonist in a first-unit dosage form, and the SRI in a second-unit dosage form, and container means for containing said first and second dosage forms.

In particular, the present invention relates to the use of, and to pharmaceutical compositions or kits comprising the following combinations:
CGP 55845 and an SRI selected from citalopram, escitalopram, fluoxetine, sertraline, paroxetine, fluvoxamine, venlafaxine, dapoxetine, vilazodone, duloxetine, nefazodone, imipramin, femoxetine and clomipramine,
CGP 62349 and an SRI selected from citalopram, escitalopram, fluoxetine, sertraline, paroxetine, fluvoxamine, venlafaxine, dapoxetine, duloxetine, vilazodone, nefazodone, imipramin, femoxetine and clomipramine,
CGP 71982 and an SRI selected from citalopram, escitalopram, fluoxetine, sertraline, paroxetine, fluvoxamine, venlafaxine, dapoxetine, duloxetine, vilazodone, nefazodone, imipramin, femoxetine and clomipramine,
CGP 76290 and an SRI selected from citalopram, escitalopram, fluoxetine, sertraline, paroxetine, fluvoxamine, venlafaxine, dapoxetine, duloxetine, vilazodone, nefazodone, imipramin, femoxetine and clomipramine,
CGP 76291 and an SRI selected from citalopram, escitalopram, fluoxetine, sertraline, paroxetine, fluvoxamine, venlafaxine, dapoxetine, duloxetine, vilazodone, nefazodone, imipramin, femoxetine and clomipramine,
CGP 35348 and an SRI selected from citalopram, escitalopram, fluoxetine, sertraline, paroxetine, fluvoxamine, venlafaxine, dapoxetine, duloxetine, vilazodone, nefazodone, imipramin, femoxetine and clomipramine,
CGP 36742 and an SRI selected from citalopram, escitalopram, fluoxetine, sertraline, paroxetine, fluvoxamine, venlafaxine, dapoxetine, duloxetine, vilazodone, nefazodone, imipramin, femoxetine and clomipramine,
CGP 46381 and an SRI selected from citalopram, escitalopram, fluoxetine, sertraline, paroxetine, fluvoxamine, venlafaxine, dapoxetine, duloxetine, vilazodone, nefazodone, imipramin, femoxetine and clomipramine,
CGP 52432 and an SRI selected from citalopram, escitalopram, fluoxetine, sertraline, paroxetine, fluvoxamine, venlafaxine, dapoxetine, duloxetine, vilazodone, nefazodone, imipramin, femoxetine and clomipramine,
CGP 54626 and an SRI selected from citalopram, escitalopram, fluoxetine, sertraline, paroxetine, fluvoxamine, venlafaxine, dapoxetine, duloxetine, vilazodone, nefazodone, imiparmin, femoxetine and clomipramine,
CGP 55845, CGP 62349, CGP 71982, CGP 76290, CGP 76291, CGP 35348, CGP 36742, CGP 46381, CGP 52432 and CGP 54626 are disclosed in Bowery NG et al. *Pharmacological Reviews* **2002**, 54 No. 2, p. 247-264.
SCH 50911 and an SRI selected from citalopram, escitalopram, fluoxetine, sertraline, paroxetine, fluvoxamine, venlafaxine, dapoxetine, duloxetine, vilazodone, nefazodone, imipramin, femoxetine and clomipramine,
Phaclofen and an SRI selected from citalopram, escitalopram, fluoxetine, sertraline, paroxetine, fluvoxamine, venlafaxine, dapoxetine, duloxetine, vilazodone, nefazodone, imipramin, femoxetine and clomipramine,
Saclofen and an SRI selected from citalopram, escitalopram, fluoxetine, sertraline, paroxetine, fluvoxamine, venlafaxine, dapoxetine, duloxetine, vilazodone, nefazodone, imipramin, femoxetine and clomipramine,
2-hydroxysaclofen and an SRI selected from citalopram, escitalopram, fluoxetine, sertraline, paroxetine, fluvoxamine, venlafaxine, dapoxetine, duloxetine, vilazodone, nefazodone, imipramin, femoxetine and clomipramine,
GAS 360 and an SRI selected from citalopram, escitalopram, fluoxetine, sertraline, paroxetine, fluvoxamine, venlafaxine, dapoxetine, duloxetine, vilazodone, nefazodone, imipramin, femoxetine and clomipramine .

In a final embodiment, the present invention relates to a method for the identification of compounds useful for the treatment of depression, anxiety disorders and other affective disorders, such as generalized anxiety disorder, panic anxiety, obsessive compulsive disorder, acute stress disorder, post traumatic stress disorder and social anxiety disorder, eating disorders such as bulimia, anorexia and obesity, phobias, dysthymia, premenstrual syndrome, cognitive disorders, impulse control disorders, attention deficit hyperactivity disorder, drug abuse or any other disorder responsive to serotonin reuptake inhibitors, comprising, in any order:
(a) measuring the ability of test compounds to inhibit serotonin reuptake and selecting the compounds that have an IC₅₀ value below 20 nM;
(b) measuring the affinity of test compounds to the GABA_{B} receptor and selecting the compounds.
and thereafter measuring the efficacy of the selected compounds at the GABA_{B} receptor and selecting the compounds which are antagonists, inverse agonists at the receptor.

Preferred GABA_{B} ligands show affinity of below 1,5 µM, whereas other preferred ligands show affinity of below 1,0 µM and yet other preferred ligands show affinity of below 500 nM. Even more preferred are compounds with affinity below 100 nM.

Examples of assays for the selection / detection of GABA_{B} antagonists, inverse agonists or partial agonists are for example the following:
Binding assay for the detection of compounds with affinity for GABA_{B} receptors are described in Karla et. al. *J*. *Med. Chem.* **1999,** 42(11), 2053-2059; or Frydenvang et. al. *Chirality* **1994**; 6(7); 583-589;
Efficacy assay for the detection of antagonists, partial agonists or inverse agonists at the GABA_{B} receptors are for example: Kamatchi et. al. *Brain Res.* **1990,** 506(2), 181-186; or Brauner-Osborne et. al. *Br. J. Pharmacol.* **1999,** 128(7), 1370-1374.

The invention also covers compounds identified according to this method, but is not limited to theses assay methods.

According to the invention, it has been found that co-administration of GABA_{B} receptor antagonist or inverse agonist and a serotonin reuptake inhibitor produces a significant increase in the level of serotonin in terminal areas, as measured in microdialysis experiments, compared to the administration of the serotonin reuptake inhibitor alone.

According to the invention, animal studies have shown that GABA_{B} receptor antagonist or inverse agonist may provide fast onset of therapeutic effect of serotonin reuptake inhibitors and potentiate the anxiolytic potential of serotonin reuptake inhibitors.

The use of a combination of GABA_{B} receptor antagonist, inverse agonist or partial agonist and a serotonin reuptake inhibitor may greatly reduce the amount of serotonin reuptake inhibitor necessary to treat depression and other affective disorders and may thus reduce the side effects caused by the serotonin reuptake inhibitor. In particular, the combination of a reduced amount of SRI and a GABA_{B} receptor antagonist, inverse agonist or partial agonist may reduce the risk of SSRI-induced sexual dysfunction and sleep disturbances.

Co-administration of a GABA_{B} receptor antagonist, inverse agonist or partial agonist and a serotonin reuptake inhibitor may also be useful for the treatment of refractory depression, i.e. depression, which cannot be treated appropriately by administration of a serotonin reuptake inhibitor alone. Typically, GABA_{B} receptor antagonist, inverse agonist or partial agonist may be used as add-on therapy for the augmentation of the response to SRIs in patients where at least 40-60% reduction in symptoms has not been achieved during the first 6 weeks of treatment with an SRI.

Compounds which are both serotonin reuptake inhibitors and GABA_{B} receptor antagonists, inverse agonists or partial agonists may have the same pharmacological advantages as the combination of a serotonin reuptake inhibitor and a GABA_{B} receptor antagonists, inverse agonists or partial agonists, with respect to reduction of side effects, fast onset and in the treatment of treatment resistant patients.

Many antidepressants with serotonin reuptake inhibiting effect have been described in the literature. Any pharmacologically active compound, which primarily or partly exert its therapeutic effect via inhibition of serotonin reuptake in the CNS, may benefit from augmentation with a GABA_{B} receptor antagonist, inverse agonist or partial agonist.

The following list contains a number of serotonin reuptake inhibitors, which may benefit from augmentation with a GABA_{B} receptor antagonist, inverse agonist or partial agonist: citalopram, escitalopram, fluoxetine, R-fluoxetine, sertraline, paroxetine, fluvoxamine, venlafaxine, desmethylvenlafaxine, duloxetine, dapoxetine, vilazodone, nefazodone, imipramine, imipramine N-oxide, desipramine, pirandamine, dazepinil, nefopam, befuraline, fezolamine, femoxetine, clomipramine, cianoimipramine, litoxetine, cericlamine, seproxetine, WY 27587, WY 27866, imeldine, ifoxetine, indeloxazine, tiflucarbine, viqualine, milnacipran, bazinaprine, YM 922, S 33005, F 98214-TA, FI 4503, A 80426, EMD 86006, NS 2389, S33005, OPC 14523, alaproclate, cyanodothepine, trimipramine, quinupramine, dothiepin, amoxapine, nitroxazepine, McN 5652, McN 5707, VN 2222, L 792339, roxindole, YM 35992, Ol 77, Org 6582, Org 6997, Org 6906, amitriptyline, amitriptyline N-oxide, nortriptyline, CL 255.663, pirlindole, indatraline, LY 280253, LY 285974, LY 113.821, LY 214.281, CGP 6085 A, RU 25.591, napamezole, diclofensine, trazodone, BMY 42.569, NS 2389, sercloremine, nitroquipazine, ademethionine, sibutramine, desmethylsubitramine, didesmethylsubitramine, clovoxamine vilazodone. The compounds mentioned above may be used in the form of the base or a pharmaceutically acceptable acid addition salt thereof. Each of the serotonin reuptake inhibitors specified above is intended to be an individual embodiment. Accordingly, each of them and the use thereof may be claimed individually.

Compounds such as citalopram, escitalopram, fluoxetine, R-fluoxetine, sertraline, paroxetine, fluvoxamine, venlafaxine, desmethylvenlafaxine, duloxetine, dapoxetine, vilazodone, nefazodone, imipramine, imipramine N-oxide, desipramine, pirandamine, dazepinil, nefopam, befuraline, fezolamine, femoxetine, clomipramine, cianoimipramine; litoxetine, cericlamine, seproxetine, imeldine, ifoxetine, indeloxazine, tiflucarbine, viqualine, milnacipran, bazinaprine, alaproclate, cyanodothepine, trimipramine, quinupramine, dothiepin, amoxapine, nitroxazepine, roxindole, amitriptyline, amitriptyline N-oxide, nortriptyline, pirlindole, indatraline, napamezole, diclofensine, trazodone, sercloremine, nitroquipazine, ademethionine, sibutramine, desmethylsubitramine, didesmethylsubitramine, clovoxamine vilazodone,
N-[(1-[(6-Fluoro-2-naphthalenyl)methyl]-4-piperidinyl]amino]carbonyl]-3-pyridine carboxamide (WY 27587),
[trans-6-(2-chlorophenyl)-1,2,3,5,6,10b-hexahydropyrrolo- (2,1-a)isoquinoline] (McN 5707),
(dl-4-exo-amino-8-chloro-benzo-(b)-bicyclo[3.3.1]nona-2-6 alpha(10 alpha)-diene hydrochloride)(Org 6997),
(dl)-(5 alpha,8 alpha,9 alpha)-5,8,9,10-Tetrahydro-5,9- methanobenzocycloocten-8-amine hydrochloride (Org 6906),
-[2-[4-(6-fluoro-1H-indol-3-yl)-3,6-dihydro-1(2H)-pyridinyl]ethyl]-3-isopropyl-6-(methylsulphonyl)-3,4-dihydro-1H-2,1,3-benzothiadiazine-2,2-dioxide (LY393558),
[4-(5,6-dimethyl-2-benzofuranyl)-piperidine] (CGP 6085), dimethyl-[5-(4-nitro-phenoxy)-6,7,8,9-tetrahydro-5H-benzocyclohepten-7-yl]-amine (RU 25.591), 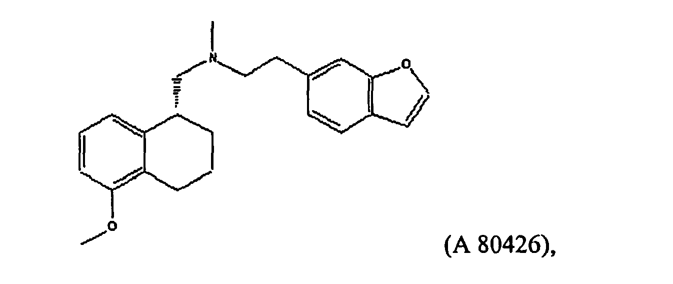 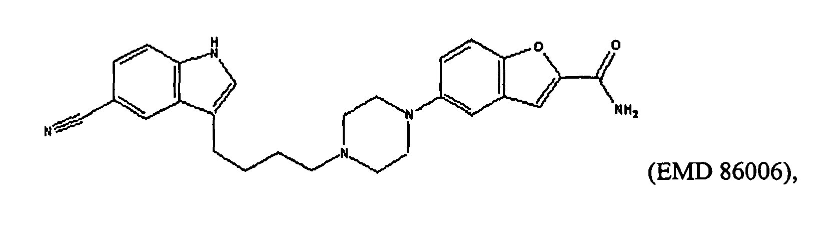 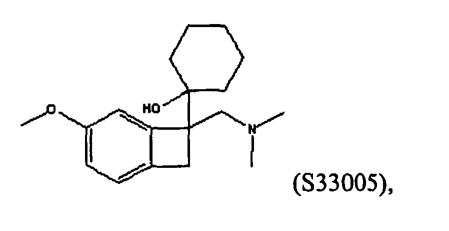 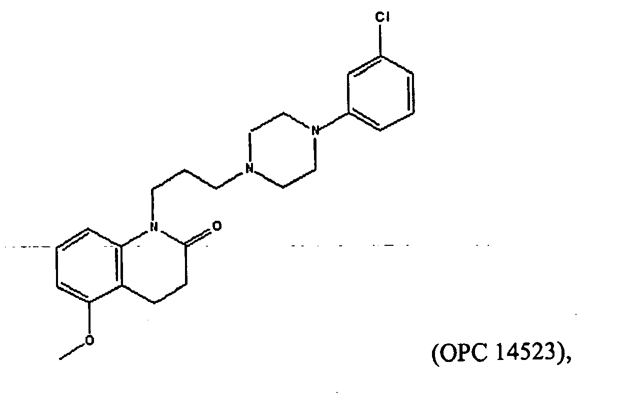 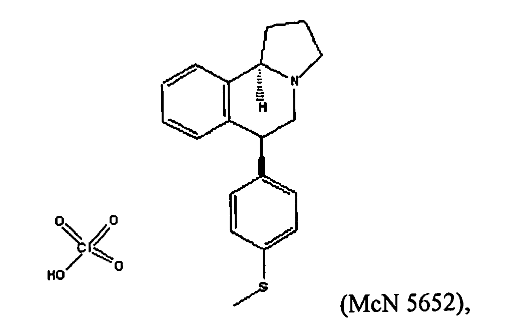 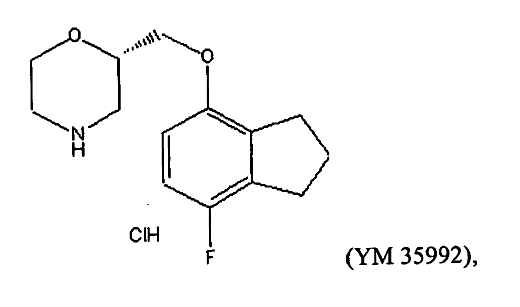 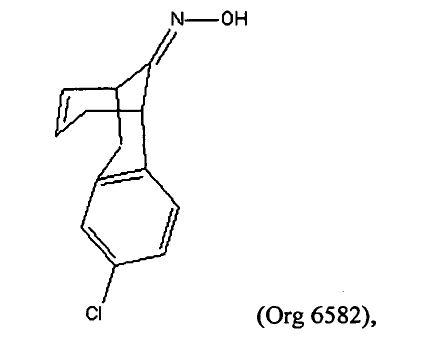
are preferred. The compounds mentioned above may be used in the form of the base or a pharmaceutically acceptable acid addition salt thereof. Each of the serotonin reuptake inhibitors specified above is intended to be an individual embodiment. Accordingly, each of them and the use thereof may be claimed individually.

Other therapeutic compounds, which may benefit from augmentation with GABA_{B} receptor antagonists, inverse agonist or partial agonists, include compounds, which cause an elevation in the extracellular level of 5-HT in the synaptic cleft, although they are not serotonin reuptake inhibitors. One such compound is tianeptine.

Accordingly, other compounds than SRIs which cause an elevation in the extracellular level of serotonin, may be used instead of SRIs in every aspect of the invention as described herein.

The above list of serotonin reuptake inhibitors and other compounds, which cause an increase in the extracellular level of serotonin, may not be construed as limiting.

SRIs, which are particularly preferred according to the present invention, include citalopram, escitalopram, fluoxetine, sertraline, paroxetine, fluvoxamine, venlafaxine, dapoxetine, duloxetine, vilazodone, nefazodone, imipramin, femoxetine and clomipramine.

The term selective serotonin reuptake inhibitor (SSRI) means an inhibitor of the monoamine transporters, which has stronger inhibitory effect at the serotonin transporter than the dopamine and the noradrenaline transporters. Particularly preferred SSRIs according to the invention are citalopram, escitalopram, fluoxetine, fluvoxamine, sertraline, duloxetine, vilnazodone and paroxetine.

In particular individual embodiments, citalopram or escitalopram is used.

The following list contains a number of GABA_{B} antagonists, partial agonists or inverse agonists, which may be used according to the invention: CGP-71982, CGP-76290, CGP-76291, CGP-35348, CGP-36742, CGP-46381, CGP-52432, CGP-54626, CGP-55845, CGP-62349, SCH 50911, GAS-360, Phaclofen, Saclofen, 2-hydroxysaclofen. Each of the GABA_{B} antagonists, partial agonists or inverse agonists specified above is intended to be an individual embodiment. Accordingly, each of them may be claimed individually.

In a preferred embodiment, a GABA_{B} receptor ligand selected from CGP 71982, CGP 76290, CGP 55845 and CGP 62349.

In particular individual embodiments, Phaclofen, 2-hydroxysaclofen or CGP-46381 is used.

Whenever mentioned, each of the terms GABA_{B} antagonist, partial agonist or inverse agonist", "GABA_{B} receptor antagonist, partial agonist or inverse agonist", "GABA_{B} ligand", and "GABA_{B} receptor ligand" means GABA_{B} receptor antagonist, partial GABA_{B} receptor agonist and inverse GABA_{B} receptor agonist. Each of which is intended to be an individual embodiment. Accordingly, each of these embodiments and the use thereof may be claimed individually.

A particular embodiment relates to a GABA_{B} receptor antagonist and the use thereof.

### Pharmaceutical compositions

Each of the active ingredients according to the invention may be administered alone or together or in combination with pharmaceutically acceptable carriers or excipients, in either single or multiple doses. The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy, 19 Edition, Gennaro, Ed., Mack Publishing Co., Easton, PA, 1995.

The pharmaceutical compositions may be specifically formulated for administration by any suitable route such as the oral, rectal, nasal, pulmonary, topical (including buccal and sublingual), transdermal, intracisternal, intraperitoneal, vaginal and parenteral (including subcutaneous, intramuscular, intrathecal, intravenous and intradermal) route, the oral route being preferred. It will be appreciated that the preferred route will depend on the general condition and age of the subject to be treated, the nature of the condition to be treated and the specific active ingredient or active ingredients chosen.

Pharmaceutical compositions for oral administration include solid dosage forms such as capsules, tablets, dragees, pills, lozenges, powders and granules. Where appropriate, they may be prepared with coatings such as enteric coatings or they may be formulated so as to provide controlled release of one or more active ingredient such as sustained or prolonged release according to methods well known in the art.

Liquid dosage forms for oral administration include solutions, emulsions, suspensions, syrups and elixirs.

Pharmaceutical compositions for parenteral administration include sterile aqueous and nonaqueous injectable solutions, dispersions, suspensions or emulsions as well as sterile powders to be reconstituted in sterile injectable solutions or dispersions prior to use. Depot injectable formulations are also contemplated as being within the scope of the present invention.

Other suitable administration forms include suppositories, sprays, ointments, cremes, gels, inhalants, dermal patches, implants etc.

The pharmaceutical compositions of this invention or those which are manufactured in accordance with this invention may be administered by any suitable route, for example orally in the form of tablets, capsules, powders, syrups, etc., or parenterally in the form of solutions for injection. For preparing such compositions, methods well known in the art may be used, and any pharmaceutically acceptable carriers, diluents, excipients or other additives normally used in the art may be used.

A typical oral dosage of each of the active ingredients is in the range of from about 0.001 to about 100 mg/kg body weight per day, preferably from about 0.01 to about 50 mg/kg body weight per day, and more preferred from about 0.05 to about 10 mg/kg body weight per day administered in one or more dosages such as 1 to 3 dosages. The exact dosage will depend upon the frequency and mode of administration, the sex, age, weight and general condition of the subject treated, the nature and severity of the condition treated and any concomitant diseases to be treated and other factors evident to those skilled in the art.

For parenteral routes such as intravenous, intrathecal, intramuscular and similar administration, typically doses are in the order of about half the dose employed for oral administration.

The compounds of this invention are generally utilized as the free substance or as a pharmaceutically acceptable salt thereof. One example is a base addition salt of a compound having the utility of a free acid. When an active ingredient contains a free acid such salts are prepared in a conventional manner by treating a solution or suspension of a free acid of the active ingredient with a chemical equivalent of a pharmaceutically acceptable base.

For parenteral administration, solutions of one or more active ingredient in sterile aqueous solution, aqueous propylene glycol, aqueous vitamin E or sesame or peanut oil may be employed. Such aqueous solutions should be suitably buffered if necessary and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art.

Solutions for injections may be prepared by dissolving one or more active ingredients and possible additives in a part of the solvent for injection, preferably sterile water, adjusting the solution to a desired volume, sterilising the solution and filling it in suitable ampules or vials. Any suitable additive conventionally used in the art may be added, such as tonicity agents, preservatives, antioxidants, etc.

Suitable pharmaceutical carriers include inert solid diluents or fillers, sterile aqueous solution and various organic solvents.

Examples of solid carriers are lactose, terra alba, sucrose, cyclodextrin, talc, agar, pectin, acacia, stearic acid and lower alkyl ethers of cellulose corn starch, potato starch, talcum, magnesium stearate, gelatine, lactose, gums, and the like.

Any other adjuvants or additives usually used for such purposes such as colourings, flavourings, preservatives etc. may be used provided that they are compatible with the active ingredient or ingredients used.

Examples of liquid carriers are syrup, peanut oil, olive oil, phospho lipids, fatty acids, fatty acid amines, polyoxyethylene and water. Similarly, the carrier or diluent may include any sustained release material known in the art, such as glyceryl monostearate or glyceryl distearate, alone or mixed with a wax.

The pharmaceutical compositions formed by combining one or more active ingredients of the invention with the pharmaceutical acceptable carriers are then readily administered in a variety of dosage forms suitable for the disclosed routes of administration. The formulations may conveniently be presented in unit dosage form by methods known in the art of pharmacy.

The active ingredients of the invention may be formulated in similar or dissimilar pharmaceurical compositions and unit forms thereof.

If a solid carrier is used for oral administration, the preparation may be tablette, placed in a hard gelatine capsule in powder or pellet form or it may be in the form of a troche or lozenge.

The amount of solid carrier will vary widely but will usually be from about 25 mg to about 1 g.

If a liquid carrier is used, the preparation may be in the form of a syrup, emulsion, soft gelatine capsule or sterile injectable liquid such as an aqueous or non-aqueous liquid suspension or solution.

If desired, the pharmaceutical composition of the invention may comprise one or more active ingredients in combination with further pharmacologically active substances such as those described in the foregoing.

### Short description of figures

*Figure 1.* Effect of local administration of bicuculline (50 µM), followed by systemic administration of citalopram 10 µmol/kg s.c.
*Figure 2.* Effect of local administration of phaclofen (50 µM), followed by systemic administration of citalopram 10 µmol/kg s.c.
*Figure 3.* Effect of co-administration of phaclofen (2 mg/kg s.c.), followed by systemic administration of citalopram (10 µmol/kg s.c.).
*Figure 4.* Effect of administration of GABA_{B} antagonist 2-hydroxysaclofen (2mg/kg s.c.) on citalopram induced increase of 5-HT levels in rat ventral hippocampus. F(1,172)=3.01, P<0.05. citalopram 10 µmol/kg s.c., n=13, citalopram 10 µmol/kg s.c. and 2-hydroxysaclofen 2 mg/kg s.c. n=3.
*Figure 5.* Effect of administration of GABA_{B} antagonist CGP 46381 (mg/kg s.c.) on citalopram induced increase of 5-HT levels in rat ventral hippocampus; citalopram 10 µmol/kg s.c., n=13, citalopram 10 µmol/kg s.c. and CGP 46381 2 mg/kg s.c. n=5; citalopram 10 µmol/kg s.c. and CGP 46381 0.5 mg/kg s.c. n=5; CGP 46381 10 mg/kg s.c. n=2.
*Figure 6.* Effect of administration of GABA_{B} antagonist Phaclofen (2mg/kg s.c.) on citalopram induced increase of 5-HT levels in rat prefrontal cortex. F(1,198)=3.25, P<0.05. citalopram 10 µmol/kg s.c., n=13, citalopram 10 µmol/kg s.c. and phaclofen 2 mg/kg s.c. n=4.

### Materials and Methods

### Animals

Male albino rats of a Wistar-derived strain (285-320 g; Harlan, Zeist, The Netherlands) were used for the experiments. Upon surgery, rats were housed individually in plastic cages (35 x 35 x 40 cm), and had free access to food and water. Animals were kept on a 12 h light schedule (light on 7:00 a.m.). The experiments are concordant with the declarations of Helsinki and were approved by the animal care committee of the faculty of mathematics and natural science of the University of Groningen.

### Drugs

The following drugs were used: Citalopram hydrobromide, 2hydroxysaclofen, CGP 46381 (Lundbeck A/S, Copenhagen, Denmark), Phaclofen and (+)-Bicuculline (Sigma, St Louis, USA).

### Surgery

Microdialysis of brain serotonin levels was performed using home made I-shaped probes, made of polyacrylonitrile / sodium methyl sulfonate copolymer dialysis fiber (i.d. 220 µm, o.d. 0.31 µm, AN 69, Hospal, Italy). Preceding surgery rats were anaesthetised using isoflurane (O₂/N₂O; 300/300ml/min). Lidocaine-HCl, 10 % (m/v) was used for local anaesthesia. Rats were placed in a stereotaxic frame (Kopf, USA), and probes were inserted into Ventral Hippcampus (V. Hippo, L +4.8 mm, IA: +3.7 mm, V: -8.0 mm) and median prefrontal cortex (PFC, L-0.9 mm; AP: +3.5 mm relative to bregma; V: -6.0 mm (Paxinos and Watson, 1982). After insertion, probes were secured with dental cement.

### Microdialysis experiments

Rats were allowed to recover for at least 24 h. Probes were perfused with artificial cerebrospinal fluid containing 147 mM NaCl, 3.0 mM KCI, 1.2 mM CaCl₂, and 1.2 mM MgCl₂, at a flow-rate of 1.5 µl/ min (Harvard apparatus, South Natick, Ma., USA). 15 minute microdialysis samples were collected in HPLC vials containing 7.5 µl 0.02 M acetic acid for serotonin analysis.

### Serotonin analysis:

Twenty-µl microdialysate samples were injected via an autoinjector (CMA/200 refrigerated microsampler, CMA, Sweden) onto a 100 x 2.0 mm C18 Hypersil 3 µm column (Bester, Amstelveen, the Netherlands) and separated with a mobile phase consisting of 5 g/L di-ammoniumsulfate, 500 mg/L EDTA, 50 mg/L heptane sulphonic acid, 4 % methanol v/v, and 30 µl/L of triethylamine, pH 4.65 at a flow of 0.4 ml/min (Shimadzu LC-10 AD). 5-HT was detected amperometrically at a glassy carbon electrode at 500 mV vs Ag/AgCl (Antec Leyden, Leiden, The Netherlands). The detection limit was 0.5 fmol 5-HT per 20 µl sample (signal to noise ratio 3).

### Data presentation and statistics

Four consecutive microdialysis samples with less then 20 % variation were taken as control and set at 100 %. Data are presented as percentages of control level (mean ± S.E.M.) in time. Statistical analysis was performed using Sigmastat for Windows (SPSS, Jandel Corporation). Treatments were compared versus controls using two way analysis of variance (ANOVA) for repeated measurements, followed by Student Newman Keuls test. Level of significance level was set at *p*<0.05.

### Results

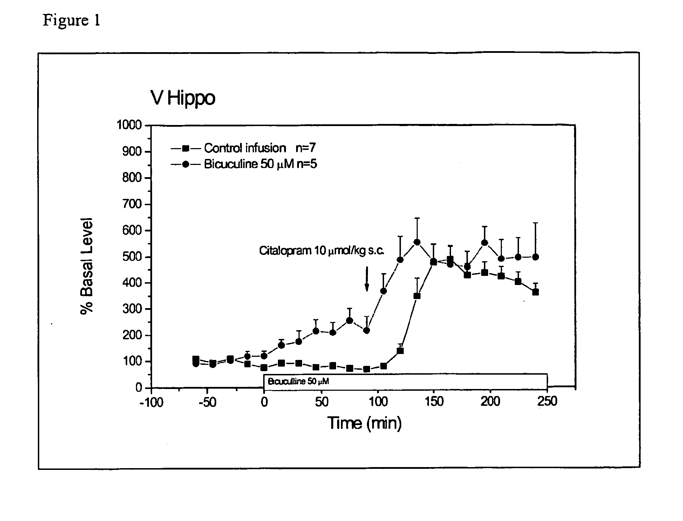

### Local administration of GABAa antagonist bicuculline, followed by systemic administration of citalopram (fig 1).

Local administration of 50 µM bicuculline in ventral hippocampus increased serotonin levels by about 150 % (treatment vs. time; F(1,79) = 5.20, P=0.0003). Post-hoc analysis revealed significance from t=45 to 90 min.
The increase established by systemic administration of 10 µmol/kg s.c. citalopram was not affected by local application of bicuculline (treatment; F(1,10) = 4.64, P= 0.0567).

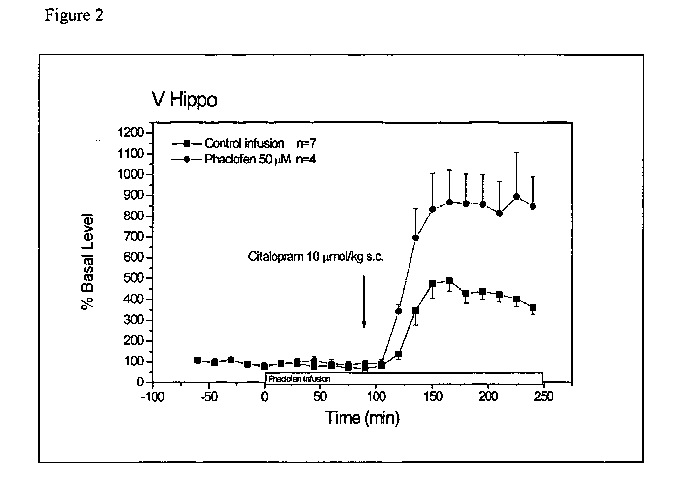

### Local administration of GABA_{B} antagonist phaclofen, followed by systemic administration of citalopram (fig 2).

Local infusion of GABA_{B} antagonist Phaclofen did not have any effect on basal levels of 5-HT in ventral hippocampus (F(1,9)= 1.44 P=0.26). Systemic administration of citalopram during local administration of phaclofen induced augmented levels of 5-HT (Treatment F(1,9)=12.21 P= 0.0068, Treatment vs. Time F(1,112) = 5.03 P<0.0001). Significant differences during post-hoc analysis was attained from t = 75 to 150 min.

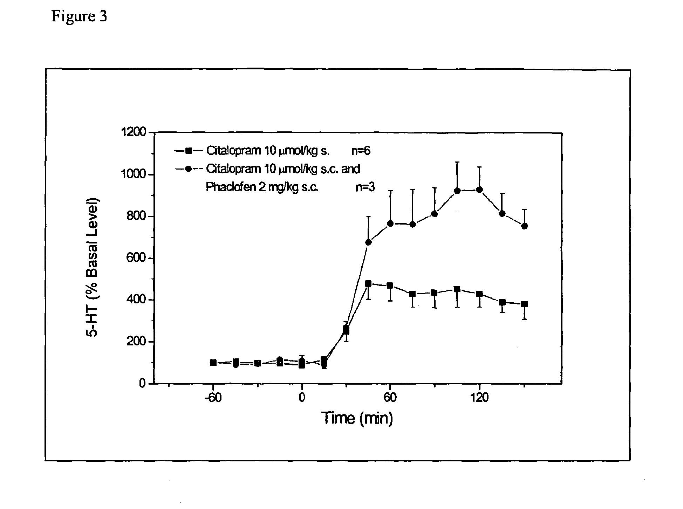

### Simultaneous administration of phaclofen 2 mg/kg s. c. with citalopram 10 µmol/kg s. c. (fig 3).

Co-administration of phaclofen 2 mg/kg s.c with citalopram 10 µmol/kg s.c. elicited enhanced levels of 5-HT when compared to citalopram treatment alone (Treatment F(1,7)=8.64 P=0.021, Treatment vs. Time, F(1,98)=6.38 P<0.0001). Post-hoc analysis showed different effects from t=75 to t=135.

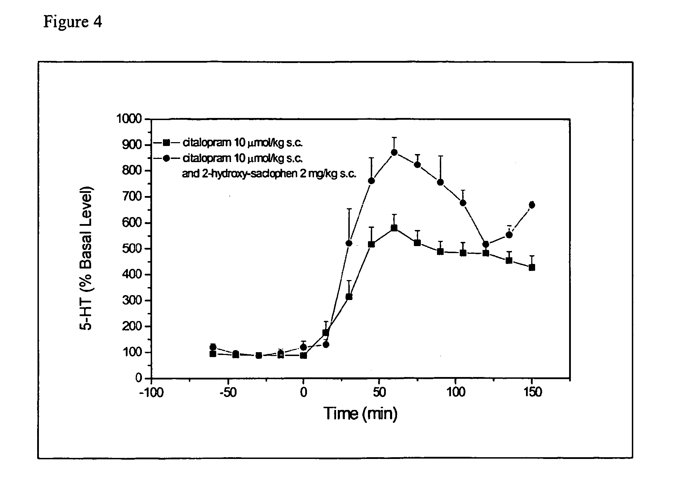

### Simultaneous administration of 2-hydroxysaclofen (2 mg/kg s.c.) with citalopram 10 µmol/kg s. c. on 5-HT levels (fig 4).

Simultaneous administration of citalopram with GABA_{B} antagonist 2-hydroxysaclofen also induced augmented effects on 5-HT levels. Treatment F(1,14)=4.80, P= 0.046, treatment versus time F(1,172)=3.01, P=0.0018.

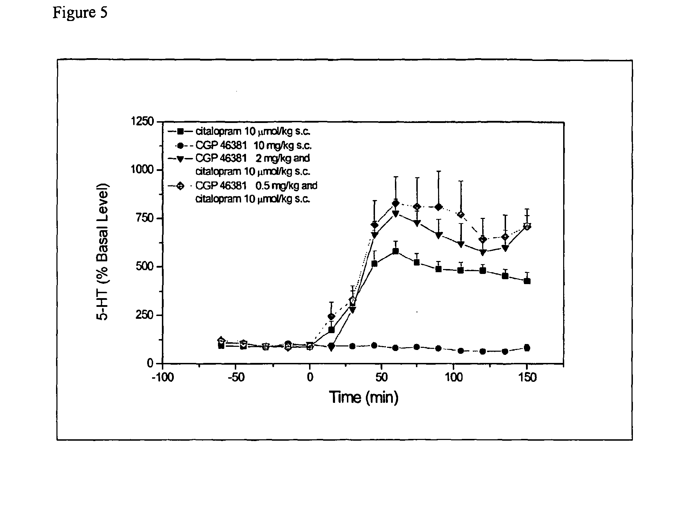

### Simultaneous administration of CGP 46381 with citalopram 10 µmol/kg s.c. on 5-HT levels (fig 5).

Administration of a high dose of the GABA_{B} antagonist CGP 46381 (10 mg/kg s.c.) did not show any effect on 5-HT levels. However, when CGP 46381 (0.5 and 2 mg/kg) was co-administered with citalopram 10 µmol/kg an augmented response on 5-HT levels was observed (0.5 mg CGP; treatment F(1,16)=4.94, p=0.04; treatment vs. time (F(1,193)= 3.24, 0.00081); 2 mg cgp treatment F(1,16)=2.94, p=0.10; treatment vs. time (F(1,192)= 3.79, 0.001)

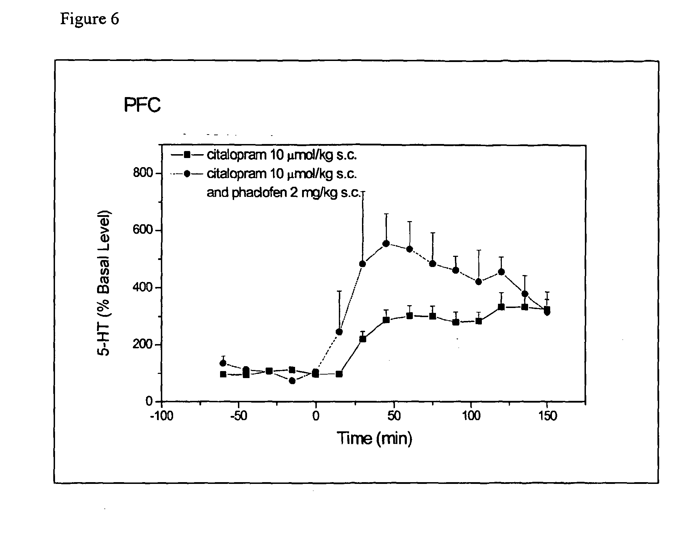

### Simultaneous administration of phaclofen 2 mg/kg s.c. with citalopram 10 µmol/kg s.c. on 5-HT levels in PFC(fig 6).

Co-administration of phaclofen 2 mg/kg s.c with citalopram 10 µmol/kg s.c. elicited enhanced levels of 5-HT when compared to citalopram treatment alone (Treatment F(1,15)=4.61 P=0.048, Treatment vs. Time, F(1,198)=6.3.25 P<0.0008).

## Claims

1. The use of a compound, which is a serotonin reuptake inhibitor, and another compound, which is a GABA_{B} receptor antagonist, inverse agonist or partial agonist for the preparation of a pharmaceutical composition for the treatment of depression, anxiety disorders and other affective disorders, such as generalized anxiety disorder, panic anxiety, obsessive compulsive disorder, acute stress disorder, post traumatic stress disorder and social anxiety disorder, eating disorders such as bulimia, anorexia and obesity, phobias, dysthymia, premenstrual syndrome, cognitive disorders, impulse control disorders, attention deficit hyperactivity disorder, drug abuse or any other disorder responsive to serotonin reuptake inhibitors.

2. The use of a GABA_{B} receptor antagonist, inverse agonist or partial agonist for the preparation of a pharmaceutical composition to be used in combination with a serotonin reuptake inhibitor.

3. The use of a GABA_{B} receptor antagonist, inverse agonist or partial agonist for the preparation of a pharmaceutical composition useful for augmenting and/or providing faster onset of the therapeutic effect of a serotonin reuptake inhibitor.

4. The use according to any of claims 2-3 wherein the serotonin reuptake inhibitor is used for the treatment of depression, anxiety disorders and other affective disorders, including generalized anxiety disorder, panic anxiety, obsessive compulsive disorder, acute stress disorder, post traumatic stress disorder or social anxiety disorder, eating disorders such as bulimia, anorexia and obesity, phobias, dysthymia, premenstrual syndrome, cognitive disorders, impulse control disorders, attention deficit hyperactivity disorder, drug abuse or any other disorder responsive to a SRI.

5. The use of a compound, which is a serotonin reuptake inhibitor, and a compound, which is a GABA_{B} receptor antagonist, inverse agonist or partial agonist,
for the preparation of a pharmaceutical composition for use in the treatment of depression, anxiety disorders and other affective disorders, such as generalized anxiety disorder, panic anxiety, obsessive compulsive disorder, acute stress disorder, post traumatic stress disorder and social anxiety disorder, eating disorders such as bulimia, anorexia and obesity, phobias, dysthymia, premenstrual syndrome, cognitive disorders, impulse control disorders, attention deficit hyperactivity disorder, drug abuse or any other disorder responsive to serotonin reuptake inhibitors.

6. The use according to any of claims 1-5 wherein a selective serotonin reuptake inhibitor is used.

7. The use according to any of claims 1-5 wherein a compound, which is selective for the GABA_{B} receptor, is used.

8. The use according to any of claims 1-5 or 7 wherein an antagonist or an inverse agonist at the GABA_{B} receptor is used.

9. The use according to claim 8 wherein a GABA_{B} receptor antagonist is used.

10. The use according to any of claims 1-6 wherein the SRI is elected from citalopram, escitalopram, fluoxetine, sertraline, paroxetine, fluvoxamine, venlafaxine, dapoxetine, duloxetine, vilazodone, nefazodone, imipramin, femoxetine and clomipramine.

11. The use according to claims 1-5 or 7-9 wherein the GABA_{B} receptor ligand is selected from CGP-71982, CGP-76290, CGP-76291, CGP-35348, CGP-36742, CGP-46381, CGP-52432, CGP-54626, CGP-55845, CGP-62349, SCH 50911, GAS-360, Phaclofen, Saclofen, 2-hydroxysaclofen.

12. A pharmaceutical composition comprising a compound, which is a serotonin reuptake inhibitor, and another compound, which is a GABA_{B} receptor antagonist, inverse agonist or partial agonist, and optionally pharmaceutically acceptable carriers or diluents.

13. A pharmaceutical composition according to claim 12 wherein the serotonin reuptake inhibitor used is a selective serotonin reuptake inhibitor.

14. A pharmaceutical composition according to claim 12 wherein the GABA_{B} antagonist, inverse agonist of partial agonist is selective for the GABA_{B} receptor.

15. A pharmaceutical composition according to any of claims 12 and 14, wherein the GABA_{B} ligand is a GABA_{B} receptor antagonist.

16. A pharmaceutical composition according to any of claims 12 and 13 **characterized in that** the serotonin uptake inhibitor is selected from citalopram, escitalopram, fluoxetine, sertraline, paroxetine, fluvoxamine, venlafaxine, dapoxetine, duloxetine, vilazodone, nefazodone, imipramin, femoxetine and clomipramine.

17. A pharmaceutical composition according to any of claims 12 and 14-15 **characterized in that** the GABA_{B} ligand is selected from CGP-71982, CGP-76290, CGP-7629I, CGP-35348, CGP-36742, CGP-46381, CGP-52432, CGP-54626,CGP-55845,CGP-55845, CGP-62349, SCH 50911, GAS-360, Phaclofen, Saclofen or 2-hydroxysaclofen.

18. A pharmaceutical composition according to any of claims 12-17, which is adapted for simultaneous administration of the active ingredients.

19. A pharmaceutical composition according to claim 18 wherein the active ingredients are contained in the same unit dosage form.

20. A pharmaceutical composition according to any of claims 12-17 which is adapted for sequential administration of the active ingredients.

21. The pharmaceutical composition according to any of claims 18 and 20 wherein the active ingredients are contained in discrete dosage forms.

22. A method for the identification of compounds useful for the treatment of depression, anxiety disorders and other affective disorders, such as generalized anxiety disorder, panic anxiety, obsessive compulsive disorder, acute stress disorder, post traumatic stress disorder and social anxiety disorder, eating disorders such as bulimia, anorexia and obesity, phobias, dysthymia, premenstrual syndrome, cognitive disorders, impulse control disorders, attention deficit hyperactivity disorder, drug abuse or any other disorder responsive to serotonin reuptake inhibitors, comprising, in any order:
(a) measuring the ability of test compounds to inhibit serotonin reuptake and selecting the compounds that have an IC₅₀ value below 20 nM;
(b) measuring the affinity of test compounds to the GARA_{A} receptor and selecting the compounds,
and thereafter measuring the efficacy of the selected compounds at the GABA_{B} receptor and selecting the compounds which are antagonists, inverse agonists or partial agonists at the receptor.

23. A method according to claim 22 wherein the compound has an affinity in step (b) of less than 500 nM;

24. A method according to any of claims 22 and 23, wherein the compound has an affinity in step (b) of less than 100 nM:

## Patentansprüche

1. Verwendung einer Verbindung, die ein Serotoninaufnahme-Hemmer ist, und eine andere Verbindung, die ein GABA_{B}-Rezeptorantagonist ist, ein inverser Agonist oder ein Teilagonist, zur Herstellung einer pharmezeutischen Zusammensetzung zur Behandlung von Depression, Angststörungen und anderen affektiven Störungen, einschließlich generalisierten Angststörungen, panischer Angst, obsessiven Zwangsstörungen, akuter Stress-Störung, posttraumatischer Stress-Störung oder sozialen Angststörungen, Ess-Störungen, wie Bulimie, Anorexia und Fettsucht, Phobie, Dysthymie, prämenstruellem Syndrom, kognitiven Störungen, Impulskontrollstörungen, Aufmerksamkeitsdefizits-Hyperaktivitäts-Störungen, Drogenmissbrauch, oder irgendwelcher anderen Störungen, die auf Serotoninaufnahme-Hemmer ansprechen.

2. Verwendung eines GABA_{B}-Rezeptorantagonisten, inversen Agonisten oder Teilagonisten zur Herstellung einer pharmazeutischen Zusammensetzung, die in Kombination mit einem Serotoninaufnahme-Hemmer verwendet werden soll.

3. Verwendung eines GABA_{B}-Rezeptorantagonisten, inversen Agonisten oder Teilagonisten zur Herstellung einer pharmazeutischen Zusammensetzung, die für die Erhöhung und/oder Bereitstellung eines schnelleren Einsetzens der therapeutischen Wirkung eines Serotoninaufnahme-Hemmers nützlich ist.

4. Verwendung gemäß irgendeinem der Ansprüche 2-3, worin der Serotoninaufnahme-Inhibitor zur Behandlung von Depression, Angststörungen und anderen affektiven Störungen, einschließlich generalisierten Angststörungen, panischer Angst, obsessiven Zwangsstörungen, akuter Stress-Störung, posttraumatischer Stress-Störung oder sozialen Angststörungen, Ess-Störungen, wie Bulimie, Anorexia und Fettsucht, Phobie, Dysthymie, prämenstruellem Syndrom, kognitiven Störungen, Impulskontrollstörungen, Aufmerksamkeitsdefizits-Hyperaktivitäts-Störungen, Drogenmissbrauch, oder irgendeine andere Störung, die auf einen SRI anspricht, verwendet wird.

5. Verwendung einer Verbindung, die einen Serotoninaufnahme-Hemmer ist, und eine Verbindung, die ein GABA_{B}-Rezeptorantagonist ist, ein inverser Agonist oder Teilagonist, zur Herstellung einer pharmazeutischen Zusammensetzung zur Verwendung bei der Behandlung von Depression, Angststörungen und anderen affektiven Störungen, einschließlich generalisierten Angststörungen, panischer Angst, obsessiven Zwangsstörungen, akuter Stress-Störung, posttraumatischer Stress-Störung oder sozialen Angststörungen, Ess-Störungen, wie Bulimie, Anorexia und Fettsucht, Phobie, Dysthymie, prämenstruellem Syndrom, kognitiven Störungen, Impulskontrollstörungen, Aufmerksamkeitsdefizits-Hyperaktivitäts-Störungen, Drogenmissbrauch, oder irgendeine andere Störung, die auf einen SRI anspricht.

6. Verwendung gemäß irgendeinem der Ansprüche 1-5, worin ein selektiver Serotoninaufnahme-Hemmer verwendet wird.

7. Verwendung gemäß irgendeinem der Ansprüche 1-5, worin eine Verbindung, die für den GABA_{B}-Rezeptor selektiv ist, verwendet wird.

8. Verwendung gemäß irgendeinem der Ansprüche 1-5 oder 7, worin ein Antagonist oder ein inverser Agonist des GABA_{B}-Rezeptors verwendet wird.

9. Verwendung gemäß Anspruch 8, worin ein GABAg-Rezeptorantagonist verwendet wird.

10. Die Verwendung gemäß irgendeinem der Ansprüche 1-6, worin der SRI ausgewählt ist aus Citalopram, Escitalopram, Fluoxetin, Sertralin, Paroxetin, Fluvoxamin, Venlafaxin, Dapoxetin, Duloxetin, Vilazodon, Nefazodon, Imipramin, Femoxetin und Clomipramin.

11. Die Verwendung gemäß Ansprüchen 1-5 oder 7-9, worin der GABA_{B}-Rezeptorligand ausgewählt ist aus CGP-71982, CGP-76290, CGP-76291, CGP-35348, CGP-36742, CGP-46381, CGP-52432, CGP-54626, CGP-55845, CGP-62349, SCH 50911, GAS-360, Phaclofen, Saclofen, 2-Hydroxysaclofen.

12. Pharmezeutische Zusammensetzung, umfassend eine Verbindung, die ein Serotoninaufnahme-Hemmer ist, und eine andere Verbindung, die ein GABA_{B}-Rezeptorantagonist, inverser Agonist oder Teilagonist ist, und gegebenenfalls pharmazeutisch annehmbare Träger und Verdünnungsmittel.

13. Pharmazeutische Zusammensetzung gemäß Anspruch 12, worin der Serotoninaufnahme-Hemmer, der verwendet wird, ein selektiver Serotoninaufnahme-Hemmer ist.

14. Pharmazeutische Zusammensetzung gemäß Anspruch 12, worin der GABA_{B}-Antagonist, inverser Agonist oder Teilagonist selektiv für den GABA_{B}-Rezeptor ist.

15. Pharmazeutische Zusammenstezung gemäß irgendeinem der Ansprüche 12 und 14, worin der GABA_{B}-Ligand ein GABA_{B}-Rezeptorantagonist ist.

16. Pharmazeutische Zusammensetzung gemäß irgendeinem der Ansprüche 12 und 13, **dadurch gekennzeichnet, dass** der Serotoninaufnahme-Hemmer ausgewählt ist aus Citalopram, Escitalopram, Fluoxetin, Sertralin, Paroxetin, Fluvoxamin, Venlafaxin, Dapoxetin, Duloxetin, Vilazodon, Nefazodon, Imipramin, Femoxetin und Clomipramin.

17. Pharmazeutische Zusammensetzung gemäß irgendeinem der Ansprüche 12 und 14-15, **dadurch gekennzeichnet, dass** der GABA_{B}-Ligand ausgewählt ist aus CGP-71982, CGP-76290, CGP-76291, CGP-35348, CGP-36742, CGP-46381, CGP-52432, CGP-54626, CGP-55845, CGP-62349, SCH 50911, GAS-360, Phaclofen, Saclofen, 2-Hydroxysaclofen.

18. Pharmazeutische Zusammensetzung gemäß irgendeinem der Ansprüche 12-17, die an die gleichzeitige Verabreichung der wirksamen Inhaltsstoffe angepasst ist.

19. Pharmazeutische Zusammensetzung gemäß Anspruch 18, worin die wirksamen Inhaltsstoffe in der gleichen Einheitsdosierungsform enthalten sind.

20. Pharmazeutische Zusammensetzung gemäß irgendeinem der Ansprüche 12-17, welche an die aufeinander folgende Verabreichung der wirksamen Inhaltsstoffe angepasst ist.

21. Pharmazeutische Zusammensetzung gemäß irgendeinem der Ansprüche 18 und 20, worin die wirksamen Inhaltsstoffe in einzelnen Dosierungsformen enthalten sind.

22. Verfahren zur Identifizierung von Verbindungen, die zur Behandlung von Depression, Angststörungen und anderen affektiven Störungen, einschließlich generalisierten Angststörungen, panischer Angst, obsessiven Zwangsstörungen, akuter Stress-Störung, posttraumatischer Stress-Störung oder sozialen Angststörungen, Ess-Störungen, wie Bulimie, Anorexia und Fettsucht, Phobie, Dysthymie, prämenstruellem Syndrom, kognitiven Störungen, Impulskontrollstörungen, Aufmerksamkeitsdefizits-Hyperaktivitäts-Störungen, Drogenmissbrauch, oder irgendeine andere Störung, die auf einen SRI anspricht, nützlich sind, umfassen in irgendeiner Reihenfolge:
(a) Messen der Fähigkeit der Testverbindungen, die Serotoninaufnahme zu inhibieren, und Auswählen der Verbindungen, die einen IC₅₀-Wert unter 20 nM haben;
(b) Messen der Affinität der Testverbindungen an den GABA_{B}-Rezeptor und Auswählen der Verbindungen,
und danach Messen der Wirksamkeit der ausgewählten Verbindungen an GABA_{B}-Rezeptor und Auswählen der Verbindungen, die Antagonisten, inverse Agonisten oder Teilagonisten des Rezeptors sind.

23. Verfahren gemäß Anspruch 22, worin die Verbindung eine Affinität in Schritt (b) von weniger als 500 nM hat.

24. Verfahren gemäß irgendeinem der Ansprüche 22 und 23, worin die Verbindung eine Affinität in Schritt (b) von weniger als 100 nM hat.

## Revendications

1. Utilisation d'un composé, qui est un inhibiteur de la réabsorption de la sérotonine, et d'un autre composé, qui est un antagoniste, un agoniste inverse ou un agoniste partiel du récepteur GABA_{B}, pour la préparation d'une composition pharmaceutique pour le traitement de la dépression, des troubles de l'anxiété et d'autres troubles affectifs, tels que le trouble d'anxiété généralisée, le trouble panique, les troubles obsessionnels compulsifs, les troubles de stress aigu, les troubles de stress post-traumatique et les troubles de phobie sociale, les troubles de l'alimentation tels que la boulimie, l'anorexie et l'obésité, les phobies, la dysthymie, le syndrome prémenstruel, les troubles cognitifs, les troubles du contrôle des impulsions, le trouble déficitaire de l'attention avec hyperactivité, la toxicomanie ou un autre trouble quelconque sensible aux inhibiteurs de la réabsorption de la sérotonine.

2. Utilisation d'un antagoniste, un agoniste inverse ou un agoniste partiel du récepteur GABA_{B}, pour la préparation d'une composition pharmaceutique à utiliser en combinaison avec un inhibiteur de la réabsorption de la sérotonine.

3. Utilisation d'un antagoniste, agoniste inverse ou agoniste partiel du récepteur GABA_{B} pour la préparation d'une composition pharmaceutique utile pour augmenter et/ou établir un démarrage plus rapide de l'effet thérapeutique d'un inhibiteur de la réabsorption de la sérotonine.

4. Utilisation selon l'une quelconque des revendications 2 et 3, dans laquelle on utilise l'inhibiteur de la réabsorption de la sérotonine pour le traitement de la dépression, des troubles de l'anxiété et d'autres troubles affectifs, y compris le trouble d'anxiété généralisée, le trouble panique, les troubles obsessionnels compulsifs, les troubles de stress aigu, les troubles de stress post-traumatique et les troubles de phobie sociale, les troubles de l'alimentation tels que la boulimie, l'anorexie et l'obésité, les phobies, la dysthymie, le syndrome prémenstruel, les troubles cognitifs, les troubles du contrôle des impulsions, le trouble déficitaire de l'attention avec hyperactivité, la toxicomanie ou un autre trouble quelconque sensible à un inhibiteur de la réabsorption de la sérotonine (SRI, serotonin reuptake inhibitor).

5. Utilisation d'un composé, qui est un inhibiteur de la réabsorption de la sérotonine, et d'un composé qui est un antagoniste, un agoniste inverse ou un agoniste partiel du récepteur GABA_{B},
pour la préparation d'une composition pharmaceutique à utiliser pour le traitement de la dépression, des troubles de l'anxiété et d'autres troubles affectifs, tels que le trouble d'anxiété généralisée, le trouble panique, les troubles obsessionnels compulsifs, les troubles de stress aigu, les troubles de stress post-traumatique et les troubles de phobie sociale, les troubles de l'alimentation tels que la boulimie, l'anorexie et l'obésité, les phobies, la dysthymie, le syndrome prémenstruel, les troubles cognitifs, les troubles du contrôle des impulsions, le trouble déficitaire de l'attention avec hyperactivité, la toxicomanie ou un autre trouble quelconque sensible aux inhibiteurs de la réabsorption de la sérotonine.

6. Utilisation selon l'une quelconque des revendications 1 à 5, dans laquelle on utilise un inhibiteur sélectif de la réabsorption de la sérotonine.

7. Utilisation selon l'une quelconque des revendications 1 à 5, dans laquelle on utilise un composé qui est sélectif pour le récepteur GABA_{B}.

8. Utilisation selon l'une quelconque des revendications 1 à 5 ou 7, dans laquelle on utilise un antagoniste ou un agoniste inverse du récepteur GABA_{B}.

9. Utilisation selon la revendication 8, dans laquelle on utilise un antagoniste du récepteur GABA_{B}.

10. Utilisation selon l'une quelconque des revendications 1 à 6, dans laquelle le SRI est choisi parmi : citalopram, escitalopram, fluoxétine, sertraline, paroxétine, fluvoxamine, venlafaxine, dapoxétine, duloxétine, vilazodone, néfazodone, imipramin, fémoxétine et clomipramine.

11. Utilisation selon les revendications 1 à 5 ou 7 à 9, dans laquelle le ligand du récepteur GABA_{B} est choisi parmi CGP-71982, CGP-76290, CGP-76291, CGP-35348, CGP-36742, CGP-46381, CGP-52432, CGP-54626, CGP-55845, CGP-62349, SCH 50911, GAS-360, phaclofen, saclofen, 2-hydroxysaclofen.

12. Composition pharmaceutique comprenant un composé, qui est un inhibiteur de la réabsorption de la sérotonine, ou un autre composé, qui est un antagoniste, un agoniste inverse ou un agoniste partiel du récepteur GABA_{B}, et éventuellement des véhicules ou diluants pharmaceutiquement acceptables.

13. Composition pharmaceutique selon la revendication 12, dans laquelle l'inhibiteur de réabsorption de la sérotonine utilisé est un inhibiteur sélectif de la réabsorption de la sérotonine.

14. Composition pharmaceutique selon la revendication 12, dans laquelle l'antagoniste, l'agoniste inverse ou l'agoniste partiel de GABA_{B} est sélectif pour le récepteur GABA_{B}.

15. Composition pharmaceutique selon l'une quelconque des revendications 12 et 14, dans laquelle le ligand de GABA_{B} est un antagoniste du récepteur GABA_{B}.

16. Composition pharmaceutique selon l'une quelconque des revendications 12 et 13, **caractérisée en ce que** l'inhibiteur de la réabsorption de la sérotonine est choisi parmi : citalopram, escitalopram, fluoxétine, sertraline, paroxétine, fluvoxamine, venlafaxine, dapoxétine, duloxétine, vilazodone, néfazodone, imipramin, fémoxétine et clomipramine.

17. Composition pharmaceutique selon l'une quelconque des revendications 12 et 14 et 15, **caractérisée en ce que** le ligand de GABA_{B} est choisi parmi CGP-71982, CGP-76290, CGP-76291, CGP-35348, CGP-36742, CGP-46381, CGP-52432, CGP-54626, CGP-55845, CGP-62349, SCH 50911, GAS-360, phaclofen, saclofen, 2-hydroxysaclofen.

18. Composition pharmaceutique selon l'une quelconque des revendications 12 à 17, qui est adaptée à l'administration simultanée des ingrédients actifs.

19. Composition pharmaceutique selon la revendication 18 dans laquelle les ingrédients actifs sont contenus dans une même forme posologique unitaire.

20. Composition pharmaceutique selon l'une quelconques des revendications 12 à 17 qui est adaptée à l'administration séquentielle des ingrédients actifs.

21. Composition pharmaceutique selon l'une quelconque des revendications 18 et 20 dans laquelle les ingrédients actifs sont contenus dans des formes posologiques distinctes.

22. Procédé pour l'identification de composés utiles pour le traitement de la dépression, des troubles de l'anxiété et d'autres troubles affectifs, tels que le trouble d'anxiété généralisée, le trouble panique, les troubles obsessionnels compulsifs, les troubles de stress aigu, les troubles de stress post-traumatique et les troubles de phobie sociale, les troubles de l'alimentation tels que la boulimie, l'anorexie et l'obésité, les phobies, la dysthymie, le syndrome prémenstruel, les troubles cognitifs, les troubles du contrôle des impulsions, le trouble déficitaire de l'attention avec hyperactivité, la toxicomanie ou un autre trouble quelconque sensible aux inhibiteurs de la réabsorption de la sérotonine, comprenant, dans un ordre quelconque :
(a) la mesure de la capacité des composés en test à inhiber la réabsorption de la sérotonine et le choix des composés qui ont une valeur de IC₅₀ inférieure à 20 nM ;
(b) la mesure de l'affinité des composés en test pour le récepteur GABA_{B} et le choix des composés,
et ensuite la mesure de l'efficacité des composés choisis sur le récepteur GABA_{B} et le choix des composés qui sont des antagonistes, des agonistes inverses ou des agonistes partiels pour le récepteur.

23. Procédé selon la revendication 22 dans lequel le composé a une affinité à l'étape (b) de moins de 500 nM.

24. Procédé selon l'une quelconque des revendications 22 et 23, dans lequel le composé a une affinité à l'étape (b) de moins de 100 nM.
